# EUROPEAN PATENT APPLICATION

(11) **EP 2 031 377 A1**
(43) Date of publication of application: **04.03.2009**
(21) Application number: 08163001.4
(22) Date of filing: 26.08.2008
(51) Int. Cl.: G01N 21/89, G01N 33/36

(54) **Automatic optical inspection apparatus for fabrics**

(30) Priority: 31.08.2007 IT UD20070151
(71) Applicant: RICAMBI TESSILI RI.TE - SpA, 33081 AVIANO (PN) (IT)
(72) Inventor: Pizzinato, Mario, 33170 Pordenone (IT)
(74) Representative: Petraz, Gilberto Luigi

(57) **Abstract**

Automatic optical inspection apparatus (10) to detect defects present in a fabric (12), comprising lighting means (20) able to illuminate a predefined portion (22) of the fabric (12). The apparatus (10) comprises at least a video acquisition device (26) able to acquire a light signal (S) arriving from the predefined portion (22) of fabric (12) illuminated by the lighting means (20). The apparatus (10) also comprises at least two mirrors (30, 32) of which a first mirror (30) of the mirrors (30, 32) is able to reflect the light signal (S) arriving from the predefined portion (22) of fabric (12). A second mirror (32) of the mirrors (30, 32) is able to reflect the light signal (S) arriving from the first mirror (30), in order to direct it toward the video acquisition device (26).

## Description

### FIELD OF THE INVENTION

The present invention concerns an automatic optical inspection apparatus for fabrics.

In particular, the apparatus according to the present invention can be used preferably but not only to detect defects present in finished fabrics and intended for use in the textile industry to make clothes, curtains or other.

### BACKGROUND OF THE INVENTION

Inspection apparatuses are known, to detect defects in finished fabrics, in which the fabric is unrolled from a first reel, subjected to visual inspection and then rolled onto a second reel.

Apparatuses are also known in which the visual inspection is carried out by means of an optical detection by a TV camera. On such apparatuses a predefined portion of fabric, tensed and moving between the two reels, is illuminated by lighting means, and the light signal emitted by the predefined illuminated portion of fabric is acquired by the TV cameras. The images acquired are processed and controlled, substantially automatically, in order to detect the presence of possible defects of the fabric, which are previously encoded in a control unit which manages the functioning of the device. A system to apply identification labels may be present, to identify the position of the defect on the fabric.

According to the width of the fabric, the optical path travelled by the light signal can be longer or shorter. The length is directly correlated to the capacity of the TV camera to effect a good detection of the defects.

One disadvantage of these known apparatuses is that, in order to be effective in detecting the defects, they are excessively cumbersome in size.

Another disadvantage is that the apparatuses are dedicated and autonomous, they comprise respective unwinding systems and rewinding systems and therefore it is difficult to adapt them to existing inspection machines or simple unwinding machines, they are cumbersome, difficult to position, move and use except by specialized personnel.

One purpose of the present invention is to achieve an optical inspection apparatus for fabrics that allows an effective control and detection of defects in the fabrics and which has a very limited size and low cost, so that it can also be applied to existing machines. In particular the apparatus according to the present invention is intended for use as an auxiliary low-cost equipment able to be assembled on existing machines, simply and quickly, while guaranteeing extremely high performance in terms of productivity and quality result.

Another purpose of the present invention is to achieve an optical inspection apparatus for fabrics that allows to detect the defects in fabrics of different widths.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, an optical inspection apparatus for the automatic detection of defects present in a fabric according to the invention comprises lighting means able to illuminate a predefined portion of the fabric to be inspected.

The apparatus according to the present invention also comprises at least a video acquisition device, able to acquire a light signal arriving from a predefined portion of fabric and illuminated by said lighting means. The video acquisition device, for example a CCD camera or similar, is also able to define, based on the light signal, at least an image indicative of the defects present in the predefined portion of fabric and is able to send the acquired image to a control and processing unit where the possible defects able to be found in the fabric are memorized and encoded.

The apparatus also comprises reflection means able to define an optical path for the light signal arriving from the predefined portion of fabric to the video acquisition device.

According to a characteristic feature of the present invention, the reflection means comprises at least two mirrors, of which a first is able to reflect the light signal arriving from the predetermined portion of fabric, and a second is able to reflect the light signal arriving from the first mirror, so as to direct it toward the video acquisition device.

According to a characteristic of the invention, the two mirrors are reciprocally positioned and directed so as to define two reflections of a substantially discordant direction, so that the relative segments of the optical path have substantially opposite directions.

In this way, by dividing the optical path of the light signal into at least three sub-paths, with a substantially zigzag development, or similar, there is a drastic reduction in the sizes of the apparatus, maintaining the overall length of the optical path of the light signal compatible with the type and size of the defects to be detected.

The apparatus also comprises a control and processing unit able to process the images produced by the video acquisition device in order to detect and signal defects in the fabric.

According to a variant of the present invention, the lighting means consists of one or more lamps of the LED type. In a preferential embodiment the LEDs of the lamps are white. In this way the fabric is illuminated by means of a homogeneous stable light component, with a predefined chromatic component, suitable for all the colors of the fabric and extremely long-lasting.

According to another variant of the present invention, the video acquisition device consists of a linear type TV camera, so as to increase the definition accuracy of the images and allow to position the TV camera, with respect to the predefined portion of fabric, at a closer distance than the distance obtained with standard TV cameras, for example with CCD sensors with a rectangular matrix. The use of the linear TV camera allows to further reduce the sizes of the apparatus.

According to another variant, the apparatus comprises a cover element able to cover the inspection zone in order to protect the light signal acquired by the TV camera both from impurities and also from environmental light.

According to another variant, the apparatus also comprises a marking device and/or to identify the position of the defect along the roll of fabric, which can-be of the labeling type, punch, ink jet or other analogous type.

According to another variant, the apparatus also comprises a device to record all the defects found, relating to type and position on the fabric of said defects, able to memorize information on memorization means, for example of the RFID type (Radio Frequency IDentification) applicable at the end of inspection on the fabric itself. This function is intended to define an "identity card" of the fabric.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a schematic representation of an apparatus according to the present invention associated with a winding-rewinding plant;
- fig. 2 is an enlarged schematic view of the apparatus in fig. 1;
- fig. 3 is a three-dimensional view of the apparatus in fig. 1;
- fig. 4 is a three-dimensional view of a variant of the apparatus in fig. 1;
- fig. 5 is a schematic representation of the functioning of the apparatus in fig. 1;
- fig. 6 is a rear view of the apparatus in fig. 1.

### DETAILED DESCRIPTION OF A PREFERENTIAL FORM OF EMBODIMENT

With reference to the attached drawings, an optical inspection apparatus 10 according to the present invention is used to detect defects in a finished fabric 12.

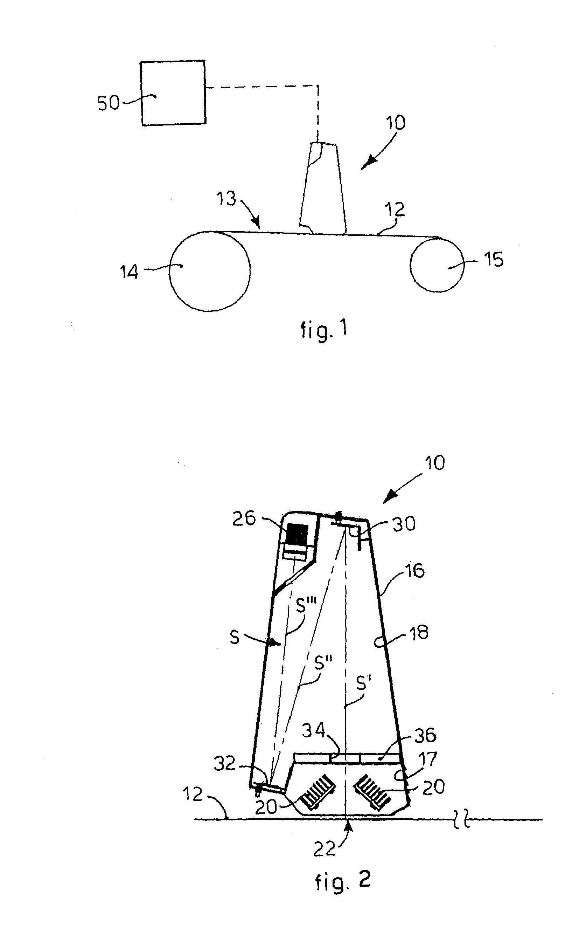

With reference to fig. 1, the apparatus 10 is applied to a manual inspection line of a winding and rewinding plant 13 of a known type, for example belonging to an already existing machine, allowing to automate the visual control operations carried out by an operator.

In particular the plant 13 comprises an unwinding device 14 able to unwind continuously a reel of fabric 12 to be inspected, and a rewinding device 15, able to rewind the fabric 12 already inspected. The fabric 12 moving at an adjustable speed, interposed between the unwinding device 14 and the rewinding device 15, is subjected to inspection by the apparatus 10.

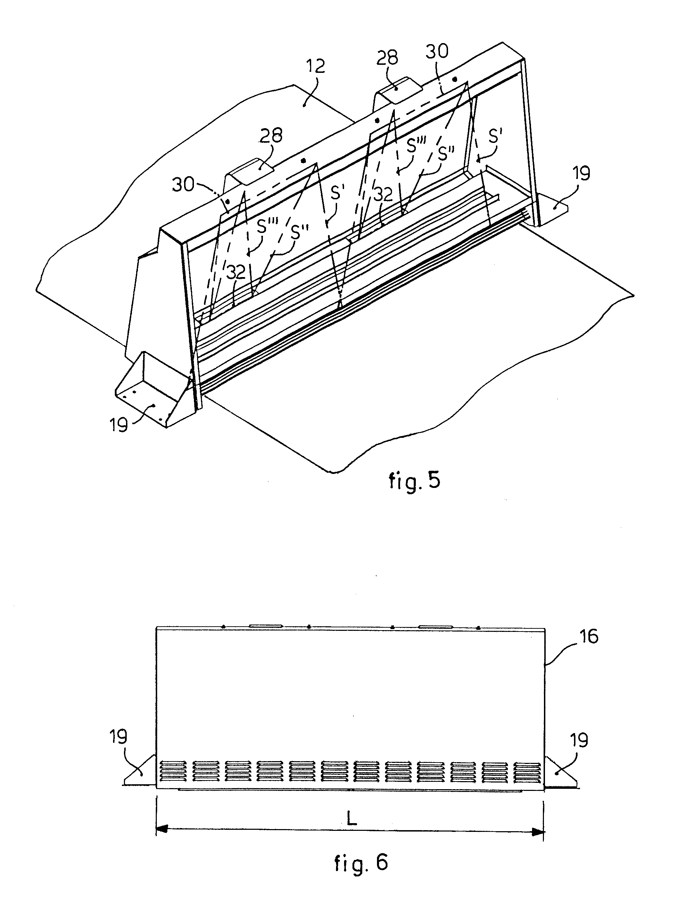

With reference to fig. 2, the apparatus 10 comprises a container 16, for example metal, advantageously made of aluminum or other material with a high capacity to dissipate heat. The inside of the container 16 is divided into a first lower compartment 17 and a second upper compartment 18 by means of a wall 36 provided with an inspection window 34 able to transmit a light signal S arriving from the predefined portion 22 of fabric 12 from the lower compartment 17 to the upper compartment 18. Inside the lower compartment 17 two lamps 20 of a known type are disposed, for example LED, able to illuminate a predefined portion 22 of fabric 12 to be inspected. The lamps 20 are disposed along the entire width L of the apparatus (fig. 6), with predefined and opposite inclinations coherent with the type of detection desired, so as to illuminate homogeneously the predefined portion 22 of fabric 22 to be inspected.

The container 16 is also provided with two brackets 19 able to be used to assemble the apparatus 10 in correspondence with the inspection line, either pre-existent or designed ex novo.

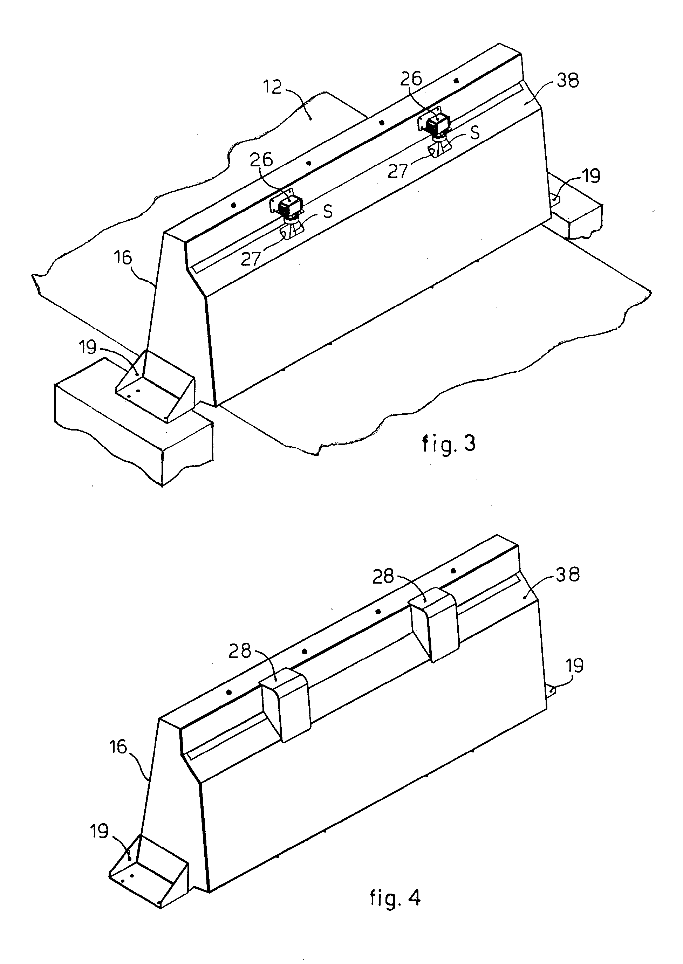

The apparatus 10 comprises in this case two TV cameras 26 of the linear type, each able to acquire a corresponding light signal S arriving from the predefined portion 22 of fabric 12 illuminated by the lamps 20. It is understood that the number of TV cameras 26 could be even more than two, or in certain cases there could be only one TV camera 26. The TV cameras 26 acquire images that, after processing, allow to detect possible defects present in the predefined portion 22 of fabric 12. Each TV camera 26 is attached (figs. 2 and 3) outside the container 16, on the upper part thereof, and receives the corresponding light signal S arriving from the compartment 18 through a corresponding window 27 disposed in the shaped wall 38 which delimits the container 16 at the upper part. Advantageously each TV camera 26 is protected both from environmental light and also from impurities by means of a cover element 28 attached to the container 16.

The apparatus 10 also comprises, inside the upper compartment 18 (figs. 2 and 5), two pairs of mirrors 30, 32, each pair associated with a corresponding TV camera 26 and able to deflect the light signal S arriving from the predefined portion 22 of fabric 12 toward the TV camera 26.

A first mirror 30 of each pair of mirrors is able to reflect the light signal S arriving through the inspection window 34 disposed along the whole width L of the apparatus between the first compartment 17 and the second compartment 18. The light signal S is emitted by the predefined portion 22 of fabric 12 in a substantially orthogonal direction with respect to the direction of movement of the fabric 12. A second mirror 32 of each pair of mirrors is able to reflect the light signal S arriving from the first mirror 30 to direct it toward the associated TV camera 26.

The mirrors 30, 32 are reciprocally disposed and oriented to define segments of the optical path which substantially change direction with every reflection. Here and in the rest of the description we mean that two segments of the optical path have a discordant direction when the angle of intersection defined by the two segments of the path is acute.

Therefore, the optical path of the signal S develops substantially in a zigzag with consecutive segments having discordant directions.

The apparatus 10 also comprises a processing unit 50 of a known type able to process the images produced by the TV cameras 26 in order to detect and signal defects in the predefined portion 22 of fabric 12 currently under inspection.

A printer, not shown in the drawings, is provided to print a summary report of the data relating to the defects found in a specific reel of fabric 12.

According to another variant, not shown in the drawings, the apparatus 10 also comprises a defect marker device, of a known type, such as for example a paper or ink jet labeler able to mark on the selvage of the fabric 12 the position of any defects found during inspection.

According to another variant, not shown in the drawings, the apparatus 10 also comprises a recorder to record all the defects detected, relating both to the type and also to the position on the fabric of the defects. The recorder memorizes the information relating to the defects on a memory with a RFID technology (Radio Frequency IDentification), applicable at the end of inspection of the fabric.

The apparatus 10 as described heretofore functions as follows.

When a new reel of fabric 12 begins to be unwound for inspection, the apparatus 10 is activated, allowing the lamps 20 to illuminate the predefined portion 22 and the TV cameras 26 to acquire the light signal S arriving by means of two consecutive reflections on the mirrors 30, 32 from the predefined portion 22 of fabric 12 illuminated by the lamps 20.

The disposition of the mirrors 30, 32 inside the container 16 is such as to divide the optical path of the light signal S by means of two consecutive reflections into three subpaths S', S" and S"', in which two consecutive segments have a substantially opposite reciprocal direction. The first subpath S' begins in the predefined portion 22 of fabric 12 and ends on the first mirror 30. The second subpath S" begins from the first mirror 30, inverting direction, and ends on the second mirror 32, and the third subpath S"' begins on the second mirror 32 and ends on the video acquisition device 26. The first subpath S' and the last subpath S"' not only have a concordant direction but are also almost parallel, that is, the angle they form is very small, in the range of some units. Thanks to the two inversions of direction and the small angle formed between consecutive segments, the size of the apparatus 10 is considerably reduced, both in height and in depth, while maintaining the overall length of the optical path relating to the light signal S, compatible with the type and size of the defects to be detected. Moreover, the first subpath S' and the last subpath S"' can intersect without compromising the formation of the image, allowing a further reduction in the depth of the apparatus 10.

The TV cameras 26 send the images produced to the processing unit 50 in order to detect the defects in the fabric 12.

When the processing unit 50 detects a defect in the fabric 12, the apparatus records the data relating to the coordinates of the position of the defect and the type of defect found, an/or prints them at the end of the inspection in the summary report of the reel of fabric 12, and/or saves them at the end of inspection on a memory, for example of the RFID type.

It is clear that modifications and/or additions of parts may be made to the apparatus 10 as described heretofore, without departing from the field and scope of the present invention.

For example the apparatus 10 can comprise a display screen, of a known type, able to display the images produced by each TV camera 26. The apparatus 10 can be provided with a video output for connection to an external display screen or to a video recording device.

It is also clear that, although the present invention has been described with reference to some specific examples, a person of skill in the art shall certainly be able to achieve many other equivalent forms of automatic optical inspection apparatus for fabrics, having the characteristics as set forth in the claims and hence all coming within the field of protection defined thereby.

## Claims

1. Automatic optical inspection apparatus to detect defects present in a fabric (12), comprising lighting means (20) able to illuminate a predefined portion (22) of said fabric (12), and at least a video acquisition device (26) able to acquire a light signal (S) arriving from said predefined portion (22) of fabric (12) illuminated by said lighting means (20), **characterized in that** it comprises at least two mirrors (30, 32) of which a first mirror (30) of said mirrors (30, 32) is able to reflect the light signal (S) arriving from said predefined portion (22) of fabric (12) and a second mirror (32) of said mirrors (30, 32) is able to reflect the light signal (S) arriving from said first mirror (30), in order to direct it toward said video acquisition device (26).

2. Apparatus as in claim 1, **characterized in that** the two mirrors (30, 32) define an optical path comprising a first subpath (S') from said predefined portion (22) of fabric (12) to said first mirror (30), a second subpath (S") from said first mirror (30) to said second mirror (32) and a third optical subpath (S"') from said second mirror (32) to said video acquisition device (26), wherein the first subpath (S') has a discordant direction from the direction of the second subpath (S").

3. Apparatus as in claim 1, **characterized in that** the two mirrors (30, 32) define an optical path comprising a first subpath (S') from said predefined portion (22) of fabric (12) to said first mirror (30), a second subpath (S") from said first mirror (30) to said second mirror (32) and a third optical subpath (S"') from said second mirror (32) to said video acquisition device (26), wherein the second subpath (S") has a discordant direction from the direction of the third subpath (S''').

4. Apparatus as in any claim hereinbefore, **characterized in that** it comprises a data processing unit (50) able to process said images produced by said video acquisition device (26).

5. Apparatus as in claim 1, **characterized in that** said lighting means comprises LED type lamps(20).

6. Apparatus as in claim 5, **characterized in that** said LEDs are white.

7. Apparatus as in one of claims 5 or 6, **characterized in that** said LED lamps (20) are disposed along the whole width (L) of the apparatus.

8. Apparatus as in any claim hereinbefore, **characterized in that** it comprises a container (16) having two compartments (17, 18) separated by a separating wall (36), of which a first compartment (17) of said two compartments (17, 18) is able to contain said lighting means (20) and a second compartment (18) of said two compartments (17, 18) is able to contain said at least two mirrors (30, 32).

9. Apparatus as in claim 8, **characterized in that** said separating wall (36) comprises an inspection window (34) able to transmit said light signal (S) arriving from said predefined portion (22) of fabric (12) illuminated by said lighting means (20),from said first compartment (17) to said second compartment (18).

10. Apparatus as in claim 9, **characterized in that** said video acquisition device comprises at least a TV camera (26) of a linear type.

11. Apparatus as in claim 10, **characterized in that** said TV camera (26) is mounted outside said container (16) on a shaped wall (38) which delimits said container (16) at the upper part.

12. Apparatus as in claim 11, **characterized in that** said shaped wall (38) comprises at least a window (27) able to transmit said light signal (S) from said second compartment (18) to said video acquisition device (26).

13. Apparatus as in claim 11, **characterized in that** it comprises at least a cover element (28) able to cover said optical path in order to protect the light signal (S) acquired by said video acquisition device (26).

14. Apparatus as in any claim hereinbefore, **characterized in that** said fabric (12) is able to move at an adjustable speed.

15. Apparatus as in any claim hereinbefore, **characterized in that** it comprises a device to record the defects, able to memorize information associated with the type and position of said defects on memorization means applied on said fabric (12).

16. Apparatus as in claim 15, **characterized in that** said memorization means comprises RFID type devices (Radio Frequency IDentification).

17. Unwinding and rewinding plant for fabric (12) comprising an optical inspection apparatus as in any claim hereinbefore.
